# EUROPEAN PATENT SPECIFICATION

(11) **EP 1 605 929 B1**
(45) Date of publication and mention of the grant of the patent: **23.04.2014**
(21) Application number: 03716776.4
(22) Date of filing: 21.03.2003
(51) Int. Cl.: A61K 31/192, A61K 31/195, A61K 31/38, A61K 31/40

(54) **NON-STEROIDAL ANTI-INFLAMMATORY DRUG DOSING REGIMEN**
DOSIERUNGSSCHEMA FÜR NICHT-STEROIDALE ANTIENTZÜNDLICHE WIRKSTOFFE
REGIME POSOLOGIQUE POUR ANTI-INFLAMMATOIRES NON STEROIDIENS

(43) Date of publication of application: 21.12.2005
(73) Proprietor: McNeil-PPC, Inc., Skillman, NJ 08558-9418 (US)
(72) Inventor: GELOTTE, Cathy, K, Blue Bell, PA 19422 (US); HOUGH, Douglas, R, Morrisville, PA 19067 (US); MCNALLY, Gerard, P., Berwyn, PA 19312 (US)
(74) Representative: Kirsch, Susan Edith
(86) International application number: PCT/US2003/008846
(87) International publication number: WO 2004/093864

(56) References cited:
- MEHLISCH, D.R.: "Double-blind crossover comparison of ketoprofen, naproxen, and placebo patients with primary dysmenorrhea" CLINICAL THERAPEUTICS, vol. 12, no. 5, 1990, pages 389-409, XP009017199
- PALMISANO, G.P. ET AL.: "Double-blind crossover comparison of ketoprofen, ibuprofen, and placebo in the treatment of patients with primary dysmanorrhea" ADVANCES IN THERAPY, vol. 5, no. 4, 1988, pages 128-137, XP009017375 cited in the application
- MEHLISCH, D.R.: "Ketoprofen, ibuprofen and placebo in the treatment of primary dysmenorrhea: a double-blind crossover comparison" J CLIN PHARMACOL., vol. 29, no. 12, December 1988 (1988-12), pages s29-33, XP009017179
- BELLAMY N (REPRINT) ET AL: "A COMPARATIVE-ANALYSIS OF 2 DOSING STRATEGIES OF FLURBIPROFEN IN RHEUMATOID-ARTHRITIS - AN APPLICATION OF SEQUENTIAL TRIAL DESIGN" CLINICAL AND INVESTIGATIVE MEDICINE-MEDECINE CLINIQUE ET EXPERIMENTALE, (1988) VOL. 11, NO. 6, PP. 441-445., XP009017116
- GREBE, W. ET AL.: "A multicenter, randomized, double-blind, double-dummy, placebo- and active- controlled, parallel-group comparison of diclofenac-K and ibuprofen for the treatment of adults with influenza-like symptoms" CLINICAL THERAPEUTICS, vol. 25, no. 2, February 2003 (2003-02), pages 444-458, XP001155030
- BLACK, P. ET AL.: "A randomized, double-blind, placebo-controlled comparison of the analgesic efficacy, onset of action, and tolerability of ibuprofen arginate and ibuprofen in postoperative dental pain" CLINICAL THERAPEUTICS, vol. 24, no. 7, 2002, pages 1072-1089, XP002254117

## Description

The present invention relates to a novel dosing regimen for non-steroidal anti-inflammatory drugs, particularly propionic acids. This dosing regimen provides sustained therapeutic effect over extended time periods.

### Background of the Invention

Therapeutic agents for treating pain, inflammation, and fever include analgesics, anti-inflammatories, and antipyretics. Non-steroidal anti-inflammatory drugs (NSAID's) are one type of such therapeutic agents. They include propionic acid derivatives, acetic acid derivatives, fenamic acid derivatives, biphenylcarbodylic acid derivatives, oxicams, and cyclooxygenase-2 (COX-2) selective NSAID's.

Propionic acids include for example ibuprofen, naproxen, and ketoprofen. Ibuprofen in particular is a widely used, well known NSAID possessing analgesic and antipyrretic properties. It has been commercially available as an over-the-counter drug in many forms for several years. Ibuprofen is chemically known as 2-(4-isobutylphenyl)-propionic acid.

NSAID's are typically administered on a once to four times daily basis, with the daily dose ranging from about 50 to about 2000 milligrams, preferably from about 100 to 1600 and most preferably from about 200 to about 1200 milligrams.

Acetaminophen is a well known analgesic, with a daily dose ranging from about 325 to about 4000 milligrams, preferably from about 650 to about 4000 milligrams. Acetaminophen was first used in medicine by Van Mering in 1893, but only since 1949 has it gained in popularity as an effective alternative to aspirin for analgesic uses in the over the counter market. The pharmacology of APAP is reviewed by B. Ameer et al., Ann. Int. Med. 87, 202 (1977). Considering the widespread use of APAP and the volume of its manufacture, both its manufacture and its use as an analgesic are well known to persons skilled in the art.

It is known to administer NSAID's, acetaminophen, and other drugs in multiple doses over 12 or 24 hours. For example, it is known to administer multiple doses containing equal amounts of ibuprofen over 12 to 24 hours. Sustained release dosage forms containing ibuprofen are also known.

Palmisano et al., Advances in Therapy, Vol. 5, No. 4, July/August 1988 reports on a study of ketoprofen and ibuprofen for treating primary dysmenorrhea. This reference discloses the use of multiple doses of ketoprofen (initial dose of 150 mg followed by subsequent doses of 75 mg) and ibuprofen (initial dose of 800 mg followed by subsequent doses of 400 mg).

WO 96/41617 describes solid oral dosage forms having an immediate release portion and a second portion which provides slow release of drug simultaneously with, or immediately following, the immediate release of drug from the dosage form.

It is useful to minimize the "drug exposure" of a patient. In other words, to administer the least total amount of drug that will provide the optimal beneficial therapeutic effect. In particular, it is useful to administer analgesics such as NSAIDs or acetaminophen in a regimen which provides maximal relief at minimal total dose per day of drug.

Applicants have now discovered that NSAID's or acetaminophen provided to a mammal, preferably a human, in a specific, two step dosing regimen provide improved therapeutic effect, especially pain relief, compared with known dosing regimens. In particular, an NSAID or acetaminophen is provided to the mammal, either in one dosage form or two dosage forms taken separately, in an initial dose followed by a second dose of about 3 to 5 hours later. No further NSAID or acetaminophen need be provided, yet surprisingly the therapeutic effect of the NSAID acetaminophen lasts at least about 6 hours after administration of the second dose.

### Summary of the Invention

The present invention provides non-steroidal anti-inflammatory drugs for use in the treatment of pain, inflammation and fever, wherein the non-steroidal anti-inflammatory drug is administered in a two-step dosing regimen said two-step dosing regimen consisting of administering an initial immediate release dose of said non-steroidal anti-inflammatory drug followed by a second immediate release dose of said non-steroidal anti-inflammatory drug about 3 to 5 hours after administration of said initial dose, wherein the first dose is twice the second dose, said non-steroidal anti-inflammatory drug having a duration of therapeutic effect which lasts at least 12 hours after administration of said second dose.

Also provided are dosage forms consisting of an immediate release portion containing an initial dose of a non-steroidal anti-inflammatory drug and a delayed burst release portion containing a second dose of said non-steroidal anti-inflammatory drug, said initial dose being twice said second dose; wherein the dissolution of the burst release portion, after the delay period, meets USP specifications for immediate release tablets containing the non-steroidal anti-inflammatory drug.

### Brief Description of the Drawings

Figure 1 depicts ibuprofen absorption levels as a function of time for the various dosing regimens reported in Example 1.
Figure 2 depicts the pain relief scores as a function of time for the dosing regimens reported in Example 1.
Figure 3 depicts the pain intensity differences (PID) as a function of time for the dosing regimens reported in Example 1.

### Detailed Description of the Invention

As used herein, "ATDAIRD" shall mean the average therapeutic duration of action of an effective immediate release dose" of a particular active ingredient. For example, the typical duration of action of an immediate release dose of ibuprofen or ketoprofen is about 4 to about 6 hours. Accordingly, the ATDAIRD for ibuprofen or ketoprofen is 5 hours. The typical duration of action of an immediate release dose of Naproxen is about 8 to about 12 hours. The ATDAIRD for naproxen, therefore is 10 hours. The therapeutic duration of action of a particular active ingredient can readily be determined from the dosing instructions in the labeling for immediate release products containing that particular active ingredient.

NSAID's useful in the present invention include for example 1) propionic acid derivative NSAID's, 2) acetic acid derivative NSAID's, 3) fenamic acid derivative NSAID's, 4) biphenylcarbodylic acid derivative NSAID's, 5) oxicam NSAID's, 6) cyclooxygenase-2 (COX-2) selective NSAID's, and 7) pharmaceutically acceptable salts of the foregoing.

Examples of acetic acid derivatives are indomethacin, diclofenac, sulindac, tolmetin, and the like. Examples of fenamic acid derivatives are mefanamic acid, meclofenamic acid, flufenamic acid, and the like. Examples of biphenylcarbodylic acid derivatives are diflunisal, flufenisal, and the like. Examples of oxicams are piroxicam, sudoxicam, isoxicam, meloxicam, and the like.

In a particularly preferred embodiment, the NSAID is selected from propionic acid derivatives. Propionic acid derivatives are pharmaceutically acceptable analgesics/non-steroidal anti-inflammatory drugs having a free -CH(CH₃)COOH or - CH₂CH₂COOH or a pharmaceutically acceptable salt group, such as -CH(CH₃)COO-Na+ or CH₂CH₂COO-Na+, which are typically attached directly or via a carbonyl functionality to a ring system, preferably an aromatic ring system.

Examples of useful propionic acid derivatives include ibuprofen, naproxen, benoxaprofen, naproxen sodium, flurbiprofen, fenoprofen, fenbuprofen, ketoprofen, indoprofen, pirprofen, carpofen, oxaprofen, pranoprofen, microprofen, tioxaprofen, suproprofen, alminoprofen, tiaprofenic acid, fluprofen and bucloxic acid.

In one embodiment of the invention, the propionic acid derivative is selected from ibuprofen, ketoprofen, flubiprofen, and pharmaceutically acceptable salts and combinations thereof.

Preferably, the propionic acid derivative is ibuprofen, 2-(4-isobutylphenyl)propionic acid, or a pharmaceutically acceptable salt thereof, such as the arginine, lysine, or histidine salt of ibuprofen. Other pharmaceutically acceptable salts of ibuprofen are described in US Patent Nos. 4,279,926, 4,873,231, 5,424,075 and 5,510,385, the contents of which are incorporated by reference.

Acetaminophen has the formula N-(4-hydroxyphenyl)acetamide and is sometimes referred to as APAP. The preparation of APAP is disclosed in-U.S. Patent No. 2,998,450.

The NSAID or acetaminophen is provided to a mammal in need of treatment, in particular pain relief treatment, in a specific dosing regimen over an extended time period, preferably over a 12 hour period. At time zero, an initial dose of the NSAID or acetaminophen is provided, i.e. administered, to the mammal. Approximately 1 ATDAIRD later, a second dose of the UNSAID or acetaminophen is provided to the mammal. After the second dose, no further NSAID or acetaminophen is administered for the remainder of the time period.

In embodiments in which ibuprofen, ketoprofen, or acetaminophen are employed as the active ingredient, the second dose is provided to the mammal approximately 3 to 5, preferably about 4, hours after administration of the first dose. In these embodiments, no further ibuprofen, ketoprofen, or acetaminophen is administered for the remainder of the 12 hour time period.

The initial dose may be for example in the range of about 0.10 to about 15 mg/kg, and the second dose may be for example in the range of about 0.05 to about 7.5 mg/kg. In one embodiment, the initial dose of NSAID or acetaminophen is at least about twice the second dose of UNSAID. In certain embodiments of the invention wherein ibuprofen is employed, the initial dose is from about 400 to about 800 mg, or from about 5 to about 12 mg/kg, and the second dose is from about 200 to about 400 mg, or from about 2.9 to about 6.0 mg/kg. In one particular embodiment of the invention wherein ibuprofen is employed, the initial dose is about 400 mg, or about 5.7 mg/kg, and the second dose is about 200 mg, or about 2.9 mg/kg. In certain other embodiments wherein ketoprofen is employed, the initial dose is from about 50 to about 100 mg, or from about 0.70 to about 1.43 mg/kg, and the second dose is from about 25 to about 50 mg, or from about 0.35 to about 0.72 mg/kg. In certain other embodiments wherein acetaminophen is employed, the initial dose is from about 650 to about 1000 mg, or from about 9.2 to about 14.3 mg/kg, and the second dose is from about 325 to about 500 mg, or from about 4.5 to about 7.2 mg/kg. Moreover, the initial dose is within the therapeutic range for the particular active ingredient employed, and is about twice the level of the second dose, which is also within the therapeutic range for the particular active ingredient employed.

The duration of the therapeutic effect of the NSAID or acetaminophen is maintained over the extended time period. In particular, the duration of therapeutic effect of the NSAID or acetaminophen lasts at least about 1.2 times the ATDAIRD for the NSAID after administration of the second dose. In particular embodiments in which the NSAID or acetaminophen has an ATDAIRD of 5 hours, e.g. embodiments in which the active ingredient is selected from ibuprofen, or ketoprofen, or acetaminophen, the duration of therapeutic effect lasts at least about 6 hours after administration of the second dose. It has been discovered that excellent pain relief in particular is maintained over extended time periods, preferably about 12 hours.

The NSAID or acetaminophen may be administered in a variety of dosage forms, for example, solid dosage forms such as tablets, capsules, liquid dosage forms such as syrups, and suspensions. The NSAID or acetaminophen may also be administered transdermally, or parenterally, such as intraveneously, intramuscularly, or subcutaneously. The NSAID or acetaminophen may also be administered rectally, for example as a suppository.

The initial and second doses may be administered together or separately. For example, if administered separately, the initial dose may be administered in a first immediate release dosage form, and the second dose may be administered about 3 to 5 hours later in a second immediate release dosage form.

In one embodiment the initial and second doses are administered in a single dosage form, preferably a single solid dosage form. For example, such a dosage form may comprise a single dosage form comprising an immediate release portion containing the initial dose of NSAID or acetaminophen and a delayed burst release portion containing the second dose of NSAID or acetaminophen. Such a single solid dosage form may be a multilayer tablet, a multiparticulate tablet, or the like. It may optionally include a barrier layer in between the two portions, for example a polymeric barrier.

As used herein, a "burst release profile" refers to a release profile which meets immediate release criteria during a specified interval. The specified interval may optionally follow a predetermined lag time. By "delayed burst release profile" it is meant that the release of at least a portion, or dose, of that particular active ingredient from the dosage form is delayed for a pre-determined time after contact with a liquid medium, such as after ingestion by the patient, and the delay period ("lag time") is followed by prompt (i.e. immediate) release of that dose of active ingredient.

In embodiments wherein the initial and second doses are provided by a dosage form that provides a delayed burst profile, the dissolution of the burst release portion of active ingredient, after the delay period, meets USP specifications for immediate release tablets containing that active ingredient. For example, for acetaminophen tablets, USP 24 specifies that in pH 5.8 phosphate buffer, using USP apparatus 2 (paddles) at 50 rpm, at least 80% of the acetaminophen contained in the dosage form is released therefrom within 30 minutes after dosing, and for ibuprofen tablets, USP 24 specifies that in pH 7.2 phosphate buffer, using USP apparatus 2 (paddles) at 50 rpm, at least 80% of the ibuprofen contained in the dosage form is released therefrom within 60 minutes after dosing. (See USP 24, 2000 Version, 19 - 20 and 856 (1999)).

The following examples further illustrate the invention, but are not meant to limit the invention in any way.

### Example 1

A double-blind, randomized, parallel, placebo-controlled, single center, PK/PD dental pain study was conducted over a 12 hour observation period to evaluate the pharmacokinetic, pharmacodynamic, efficacy and safety profiles of certain ibuprofen dosing regimens. Specifically, a single dose of 600 mg ibuprofen extended release caplets was compared with equivalent total doses of ibuprofen immediate release 200 mg caplets administered in three different dosing regimens as well as placebo in the treatment of moderate to severe post-operative dental pain.

The ibuprofen was administered as ibuprofen extended release 600 mg caplets or one or more ibuprofen immediate release 200 mg caplets.

The patients evaluated in this study consisted of male or non-pregnant and non-lactating female out-patient volunteers, 16 years of age or older, complaining of moderate to severe pain following the surgical extraction of three or four third molars with at least one partial or complete bony impacted third mandibular molar. The term impacted included: partial bony impaction, bony impaction, or complicated bony impaction. A total of 210 patients were entered into the study. 208 patients were eligible for the efficacy analyses.

All patients who met the entrance criteria were enrolled in one of two study sub-groups. One sub-group of patients had both pharmacokinetic and pharmacodynamic evaluation (PK group). The other sub-group of patients had only the analgesic efficacy evaluations (non-PK group). A separate randomization schedule was used for each of the two sub-groups. Patients from both subgroups were assigned at random to one of the five following treatments:

| | |
|---|---|
| Ibuprofen Extended Release | 600 mg single dose at 0 hour |
| Ibuprofen Immediate Release | 600 mg single dose at 0 hour |
| Ibuprofen Immediate Release | 400 mg at 0 hour; 200 mg at 4 hours |
| Ibuprofen Immediate Release | 200 mg at 0, 4, and 8 hours |
| Placebo | |

Patients' assessments of pain intensity and pain relief as well as blood samples for plasma ibuprofen analysis were obtained at the study site at hours 0, 0.25, 0.5, 1, 1.5, 2, 2.5, 3, 4, 4.5, 5, 6, 7, 8, 8.5, 9, 10, 11 and 12. A stopwatch technique was used to measure the onset of meaningful pain relief.

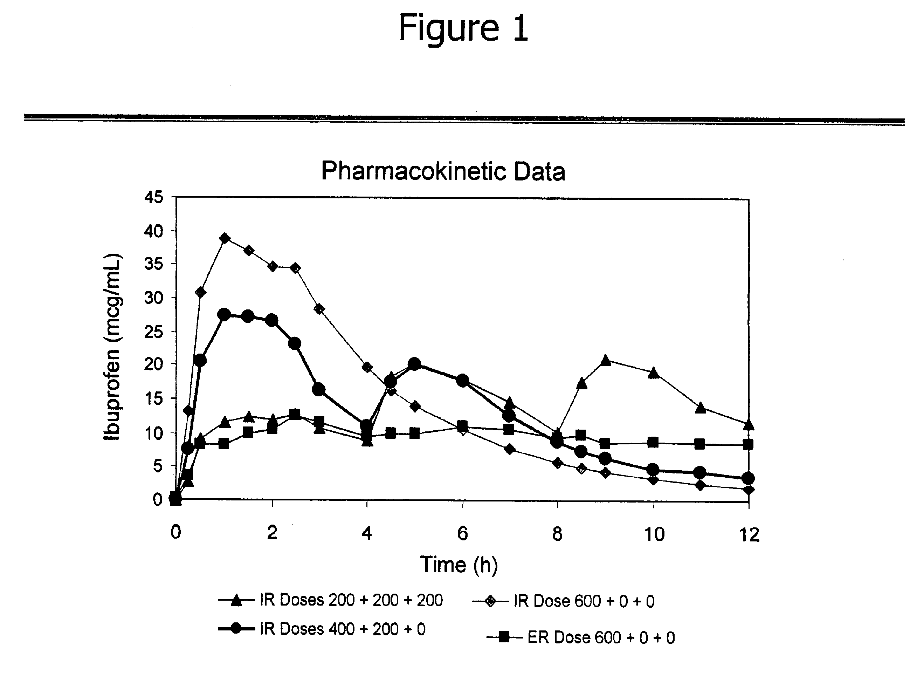

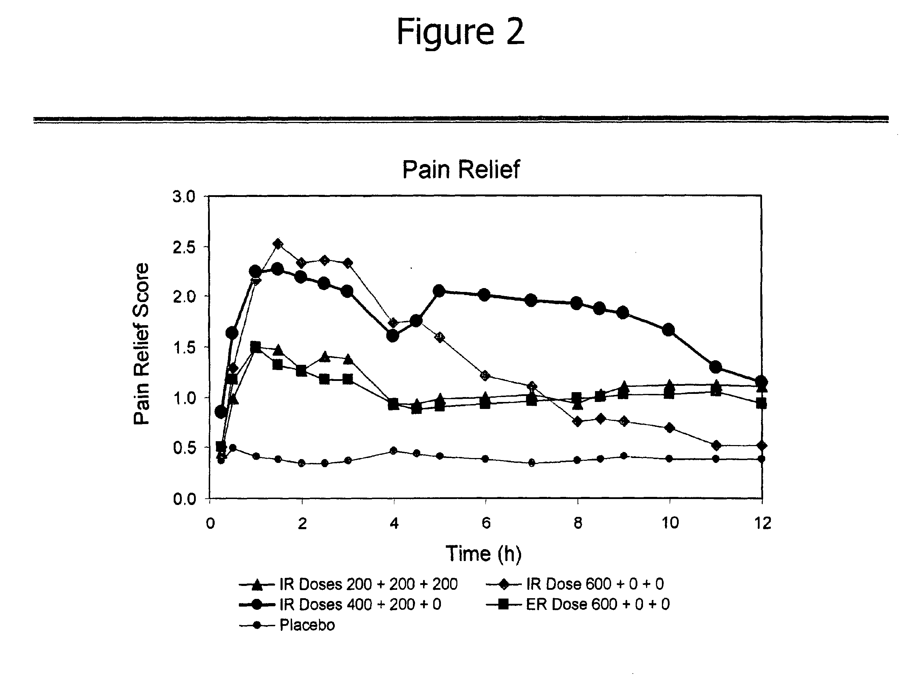

Figure 1 shows the blood levels of ibuprofen achieved with the four non-placebo treatments as a function of time over the 12 hour study period. Figure 2 depicts the pain relief scores as a function of time for the five treatments including placebo. Surprisingly, the 400/200/0 administration of ibuprofen according to the invention provided excellent pain relief over the entire 12 hour study period. During the first four hours, the 400/200/0 treatment, along with the 600/0/0 immediate release treatment, provided superior pain relief over the other treatments. During the 4 to 12 hour interval, the 400/200/0 treatment alone provided the highest pain relief scores, despite the fact that no further ibuprofen was administered after the first four hours.

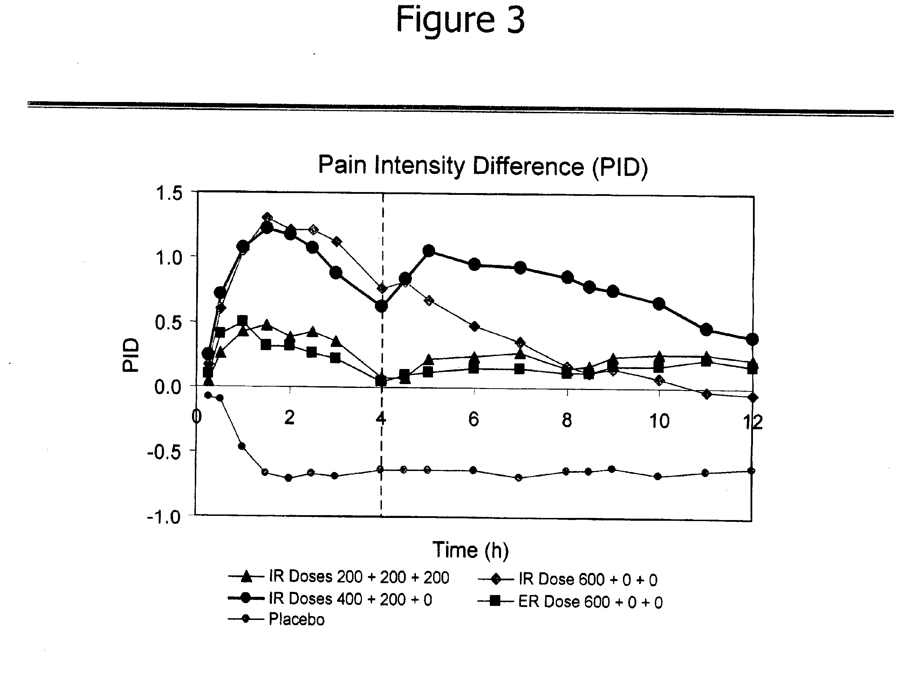

Similarly, Figure 3 shows the pain intensity differences (PID) reported as a function of time for all five treatments. Again, the 400/200/0 treatment according to the invention provided the best PID over the 12 hour study period, particularly after the four hour mark, when the PID's reported for other treatments declined. This is surprising in that the treatment according to the invention did not include further administration of ibuprofen after hour four.

### Example 2

A dosage form according to the invention is made as follows. The dosage form is an immediate release/delayed burst release combination capsule containing 200mg and 100mg of ibuprofen, respectively. The immediate release portion is in the form of granules, while the delayed burst release portion is in the form of a compression-coated core.

### STEP A

First, an ibuprofen granulation is prepared from the following ingredients:

| Ingredient | Trade Name | Manufacturer | Mg/Tablet |
|---|---|---|---|
| Ibuprofen powder | | Albemarle Corp. Orangeburg, SC | 100 |
| Microcrystalline cellulose | Avicel pH 101 | FMC Corp. Newark, DE 19711 | 5.4 |
| Pregelatinized starch | National 1551 | National Starch & Chem. Co. Bridgewater, NJ | 2.2 |
| Magnesium stearate | | Mallinckrodt Inc., St. Louis, Missouri | 1.1 |
| Total | | | 108.7 |

The ibuprofen powder, AVICEL pH 101 and pregelatinized starch are mixed in a (5qt) bowl of a planetary mixer (Hobart Corp., Dayton, OH). Water is added to the powder mixture while mixing at low speed. Mixing is continued for 10 minutes. The granulation is removed from the bowl and dried at room temperature for 12 to 16 hours to remove all residual solvent. The granules are screened through a #20 mesh screen and placed in a (2 qt) P-K blender. Magnesium stearate is added to the dry granules, followed by mixing for 5 more minutes.

### STEP B

The granulation of Step A is compressed into cores on a Beta Press (Manesty, Liverpool, UK). The press is equipped with 6 mm diameter round, concave punch and die units. The granulation is fed into the die cavity of the press and pressed into solid cores using 1500 lb/sq. in. of operating pressure. The compressed cores weigh 109 mg and contained 100 mg of ibuprofen.

### STEP C

An ethylcellulose powder is prepared as a compression-coating for the sustained burst release portion of the dosage form. The following ingredients are used:

| Ingredient | Manufacturer | Mg/Tablet |
|---|---|---|
| Ethylcellulose powder (grade N-10-F) | Shin-Etsu Chem. Ind. Co. Ltd. Tokyo, Japan | 304.6 |
| Magnesium stearate | Mallinckrodt Inc., St. Louis, Missouri | 1.4 |
| Total | | 306 |

The ethylcellulose powder and magnesium stearate are placed in a (2-quart) P-K blender and mixed for 5 minutes.

### STEP D

Compression-coated cores are prepared by using a model M hydraulic Carver Laboratory Press (Fred S. Carver, Inc., Hydraulic Equipment, Summit, NJ). The press is equipped with 9 mm round, concave punch and die units. Each core is prepared by first filling 153 mg of the ethylcellulose powder from Step C into a die, and then manually placing a core from Step B in the center of the powder. The remaining 153mg of ethylcellulose powder is then poured into the die, which is then compressed at 3000 lb/square inch of operating pressure to prepare the compression-coated core. The compression-coated cores weigh 415 mg and contain 100 mg of ibuprofen.

### STEP E

The finished dosage form comprising immediate release and delayed burst release portions of ibuprofen is made as follows. 218 mg of the ibuprofen granulation from Step A is filled into the first half of a capsule (DB Caps, size AA, Capsugel, Morris Plains, NJ). Next, a compression-coated core of Step D is manually placed into the capsule. The second half of the capsule is then inserted into the first half of the capsule. The finished dosage form contains 218 mg of ibuprofen granulation (equivalent to 200 mg of ibuprofen) as the immediate release portion of ibuprofen and a 415 mg compression-coated ibuprofen core (equivalent to 100 mg of ibuprofen) as the delayed burst release dose of the ibuprofen.

### Example 3

A dosage form according to the invention is made as follows. The dosage form is an immediate release/delayed burst release combination capsule containing 200mg and 100mg of ibuprofen, respectively. The immediate release portion is in the form of granules, while the delayed burst release portion is in the form of a spray-coated core.

### STEP A

An ibuprofen granulation for the immediate release portion is prepared as in Example 2 above.

### STEP B

The granulation of Step A is compressed into cores as in Example 2 above.

### STEP C

A coating dispersion for the sustained burst release portion of the dosage form is prepared using the following ingredients:

| Coating | Trade Name | Manufacturer | Mg/Tablet |
|---|---|---|---|
| Hydroxypropyl methylcellulose | Methocel K4M | The Dow Chemical Company, Midland, Michigan 48674 | 20.8 |
| Talc | | Charles B. Chrystal Co. New York, NY | 8.3 |
| Povidone | Kollidon K-30 | BASF Corp. Parsipany, NJ | 10.4 |
| Polyethylene Glycol 400 | | Union Carbide Corporation, Danbury, CT 06817 | 4.1 |
| Ethanol (dried as solvent) | | | |
| Water (dried as solvent) | | | |
| Total | | | 43.6 |

The coating dispersion is prepared by adding the hydroxypropyl methylcellulose, talc, povidone, and polyethylene glycol 400 to a suitable ethanol/water mixture (94%/4%) to produce a 10.5% polymeric dispersion. The dispersion is allowed to sit at room temperature for 12 hours.

### STEP D

Spray-coated ibuprofen cores are prepared as follows. The cores of Step B are placed in a 24" Acella Coating pan (Manesty, Liverpool, UK) and air tumbled with the coating dispersion of Step C until the cores are uniformly coated. The coated cores are dried in an oven at 50°C for 24 hours to evaporate the solvent. The coated cores weigh 153 mg and contain 100 mg of ibuprofen.

### STEP E

The finished dosage form comprising immediate release and delayed burst release portions of ibuprofen is made as follows. 218 mg of the ibuprofen granulation of Step A is filled into a first half of a capsule (DB Caps, size AA, Capsugel, Morris Plains, NJ). A spray-coated ibuprofen core of Step D is manually placed into the capsule. The second half of the capsule is then inserted into the first half to yield the finished dosage form. The finished dosage form contains 218 mg of ibuprofen granulation (equivalent to 200 mg of ibuprofen) as the immediate release portion of the ibuprofen and a 153 mg spray-coated ibuprofen core (equivalent to 100 mg of ibuprofen) as the delayed burst release portion of ibuprofen.

## Claims

1. A non-steroidal anti-inflammatory drug for use in the treatment of pain, inflammation and fever, wherein the non-steroidal anti-inflammatory drug is administered in a two-step dosing regimen said two-step dosing regimen consisting of administering an initial immediate release dose of said non-steroidal anti-inflammatory drug followed by a second immediate release dose of said non-steroidal anti-inflammatory drug about 3 to 5 hours after administration of said initial dose, wherein the first dose is twice the second dose, said non-steroidal anti-inflammatory drug having a duration of therapeutic effect which lasts at least 12 hours after administration of said second dose.

2. The non-steroidal anti-inflammatory drug for use according to claim 1, wherein said non-steroidal anti-inflammatory drug is a propionic acid derivative and said propionic acid derivative is selected from the group consisting of ibuprofen, naproxen, benoxaprofen, naproxen sodium, flurbiprofen, fenoprofen, fenbuprofen, ketoprofen, indoprofen, pirprofen, carpofen, oxaprofen, pranoprofen, microprofen, tioxaprofen, suproprofen, alminoprofen, tiaprofenic acid, fluprofen and bucloxic acid.

3. A dosage form consisting of an immediate release portion containing an initial dose of a non-steroidal anti-inflammatory drug and a delayed burst release portion containing a second dose of said non-steroidal anti-inflammatory drug, said initial dose being twice said second dose; wherein the dissolution of the burst release portion, after the delay period, meets USP specifications for immediate release tablets containing the non-steroidal anti-inflammatory drug.

4. The dosage form of claim 3, wherein said non-steroidal anti-inflammatory drug is a propionic acid derivative.

5. The dosage form of claim 3, wherein said propionic acid derivative is selected from the group consisting of ibuprofen, naproxen, benoxaprofen, naproxen sodium, flurbiprofen, fenoprofen, fenbuprofen, ketoprofen, indoprofen, pirprofen, carpofen, oxaprofen, pranoprofen, microprofen, tioxaprofen, suproprofen, alminoprofen, tiaprofenic acid, fluprofen and bucloxic acid.

6. The dosage form of claim 5, wherein said propionic acid derivative is ibuprofen.

7. The dosage form of claim 3, wherein said dosage form is a solid dosage form.

8. The dosage form of claim 3, wherein said initial dose is about 5 to about 12mg/kg and said second dose is about 2.9 to about 6 mg/kg.

9. The non-steroidal anti-inflammatory drug for use according to claim 1, wherein said non-steroidal anti-inflammatory drug is acetaminophen.

## Patentansprüche

1. Nichtsteroidaler entzündungshemmender Arzneistoff zur Verwendung bei der Behandlung von Schmerzen, Entzündungen und Fieber, wobei der nichtsteroidale entzündungshemmende Arzneistoff in einem zweistufigen Dosierungsschema verabreicht wird, das aus dem Verabreichen einer Erstdosis des nichtsteroidalen entzündungshemmenden Arzneistoffs mit sofortiger Freisetzung, gefolgt von einer zweiten Dosis des nichtsteroidalen entzündungshemmenden Arzneistoffs mit sofortiger Freisetzung etwa 3 bis 5 Stunden nach Verabreichung der Erstdosis besteht, wobei die erste Dosis das Doppelte der zweiten Dosis beträgt, wobei der nichtsteroidale entzündungshemmende Arzneistoff eine therapeutische Wirkungsdauer aufweist, die nach Verabreichung der zweiten Dosis mindestens 12 Stunden lang anhält.

2. Nichtsteroidaler entzündungshemmender Arzneistoff zur Verwendung gemäß Anspruch 1, wobei es sich bei dem nichtsteroidalen entzündungshemmenden Arzneistoff um ein Propionsäurederivat handelt und das Propionsäurederivat aus der aus Ibuprofen, Naproxen, Benoxaprofen, Naproxen-Natrium, Flurbiprofen, Fenoprofen, Fenbuprofen, Ketoprofen, Indoprofen, Pirprofen, Carpofen, Oxaprofen, Pranoprofen, Microprofen, Tioxaprofen, Suproprofen, Alminoprofen, Tiaprofensäure, Fluprofen und Bucloxinsäure bestehenden Gruppe ausgewählt ist.

3. Dosierungsform, bestehend aus einem Anteil zur sofortigen Freisetzung, der eine Erstdosis eines nichtsteroidalen entzündungshemmenden Arzneistoffs enthält, und einem Anteil zur verzögerten plötzlichen Freisetzung, der eine zweite Dosis des nichtsteroidalen entzündungshemmenden Arzneistoffs enthält, wobei die Erstdosis das Doppelte der zweiten Dosis beträgt, wobei die Auflösung des Anteils zur plötzlichen Freisetzung nach der Verzögerungszeit USP-Vorgaben für den nichtsteroidalen entzündungshemmenden Arzneistoff enthaltende Tabletten mit sofortiger Freisetzung erfüllt.

4. Dosierungsform nach Anspruch 3, wobei es sich bei dem nichtsteroidalen entzündungshemmenden Arzneistoff um ein Propionsäurederivat handelt.

5. Dosierungsform nach Anspruch 3, wobei das Propionsäurederivat aus der aus Ibuprofen, Naproxen, Benoxaprofen, Naproxen-Natrium, Flurbiprofen, Fenoprofen, Fenbuprofen, Ketoprofen, Indoprofen, Pirprofen, Carpofen, Oxaprofen, Pranoprofen, Microprofen, Tioxaprofen, Suproprofen, Alminoprofen, Tiaprofensäure, Fluprofen und Bucloxinsäure bestehenden Gruppe ausgewählt ist.

6. Dosierungsform nach Anspruch 5, wobei es sich bei dem Propionsäurederivat um Ibuprofen handelt.

7. Dosierungsform nach Anspruch 3, bei der es sich um eine feste Dosierungsform handelt.

8. Dosierungsform nach Anspruch 3, wobei die Erstdosis etwa 5 bis etwa 12 mg/kg und die zweite Dosis etwa 2,9 bis etwa 6 mg/kg beträgt.

9. Nichtsteroidaler entzündungshemmender Arzneistoff zur Verwendung gemäß Anspruch 1, wobei es sich bei dem nichtsteroidalen entzündungshemmenden Arzneistoff um Acetaminophen handelt.

## Revendications

1. Médicament anti-inflammatoire non stéroïdien destiné à être utilisé dans le traitement de la douleur, de l'inflammation et de la fièvre, dans lequel le médicament anti-inflammatoire non stéroïdien est administré dans un régime posologique en deux étapes, ledit régime posologique en deux étapes consiste à administrer une dose initiale à libération immédiate dudit médicament anti-inflammatoire non stéroïdien suivie d'une deuxième dose à libération immédiate dudit médicament anti-inflammatoire non stéroïdien environ 3 a 5 heures après l'administration de ladite dose initiale, la première dose étant le double de la deuxième dose, ledit médicament anti-inflammatoire non stéroïdien possédant une durée d'effet thérapeutique qui dure au moins 12 heures après l'administration de ladite deuxième dose.

2. Médicament anti-inflammatoire non stéroïdien destiné à être utilisé selon la revendication 1, dans lequel ledit médicament anti-inflammatoire non stéroïdien est un dérivé d'acide propionique et ledit dérivé d'acide propionique est choisi parmi le groupe constitué par l'ibuprofène, le naproxène, le bénoxaprofène, le naproxène sodique, le flurbiprofène, le fénoprofène, le fenbuprofène, le kétoprofène, l`indoprofène, le pirprofène, le carprofène, l`oxaprofène, le pranoprofène, le microprofène, le tioxaprofène, le suproprofène, l'alminoprofène, l'acide tiaprofénique, le fluproféne et l'acide bucloxique.

3. Forme de dosage composée d'une portion à libération immédiate contenant une dose initiale d'un médicament anti-inflammatoire non stéroïdien et une portion à libération brutale retardée contenant une deuxième dose dudit médicament anti-inflammatoire non stéroïdien, ladite dose initiale étant le double de la deuxième dose ; la dissolution de la portion à libération brutale, après la période de retard, étant conforme aux spécifications de l'USP pour les tablettes à libération immédiate contenant le médicament anti-inflammatoire non stéroïdien.

4. Forme de dosage selon la revendication 3, dans laquelle le médicament anti-inflammatoire non stéroïdien est un dérivé de l'acide propionique.

5. Forme de dosage selon la revendication 3, dans laquelle ledit dérivé de l'acide propionique est choisi parmi le groupe constitué par l'ibuprofène, le naproxène, le bénoxaprofène, le naproxène sodique, le flurbiprofène, le fénoprofène, le fenbuprofène, le kétoprofène, l'indoprofène, le pirprofène, le carprofène, l'oxaprofène, le pranoprofène, le microprofène, le tioxaprofène, le suproprofène, l'alminoprofène, l'acide tiaprofénique, le fluprofène et l'acide bucloxique.

6. Forme de dosage selon la revendication 5, dans laquelle ledit dérivé de l'acide propionique est l`ibuprofène.

7. Forme de dosage selon la revendication 3, dans laquelle ladite forme de dosage est une forme de dosage solide.

8. Forme de dosage selon la revendication 3, dans laquelle ladite dose initiale est d'environ 5 à environ 12 mg/kg et ladite deuxième dose est d'environ 2,9 à environ 6 mg/kg.

9. Médicament anti-inflammatoire non stéroïdien destiné à être utilisé selon la revendication 1, dans lequel ledit médicament anti-inflammatoire non stéroïdien est l'acétaminophène.
